# FASCICULE DE BREVET EUROPEEN

(11) **EP 4 076 263 B1**
(45) Date de publication et mention de la délivrance du brevet: **10.07.2024**
(21) Numéro de dépôt: 20845208.6
(22) Date de dépôt: 15.12.2020
(51) Int. Cl.: A61B 50/20, A61B 50/30, A61F 2/00

(54) **ENSEMBLE COMPRENANT UN DISPOSITIF MEDICAL ADAPTE POUR ETRE IMPLANTE ET UN EMBALLAGE DUDIT DISPOSITIF**
ANORDNUNG MIT EINER IMPLANTIERBAREN MEDIZINISCHEN VORRICHTUNG UND VERPACKUNG FÜR DIESE VORRICHTUNG
ASSEMBLY COMPRISING A MEDICAL DEVICE ADAPTED TO BE IMPLANTED AND PACKAGING FOR SAID DEVICE

(30) Priorité: 16.12.2019 FR 1914528
(43) Date de publication de la demande: 26.10.2022
(73) Titulaire: Uromems, 38320 Eybens (FR)
(72) Inventeur: LAMRAOUI, Hamid, 38410 Vaulnaveys le Haut (FR); MOZER, Pierre, 94300 VINCENNES (FR); BEAUGERIE, Aurélien, 92130 ISSY LES MOULINEAUX (FR); MIR, Riaz, 38600 FONTAINE (FR); HO, Thierry, 38120 Saint Egrève (FR)
(74) Mandataire: Regimbeau
(86) Numéro de dépôt international: PCT/FR2020/052449
(87) Numéro de publication internationale: WO 2021/123606

(56) Documents cités:
- US-A- 4 726 404
- US-A1- 2012 103 840
- US-A1- 2015 021 221
- US-A1- 2017 056 149

## Description

### Domaine technique

L'invention concerne un ensemble comprenant un dispositif médical adapté pour être implanté dans un corps humain ou animal comprenant un réservoir de fluide, et un emballage dudit dispositif.

### Etat de la technique

Divers dispositifs médicaux implantables comprennent un réservoir de fluide biocompatible couplé à un circuit fluidique. Un tel circuit fluidique peut par exemple permettre d'actionner un élément du dispositif.

Par exemple, un dispositif pour obturer sélectivement un conduit anatomique, tel que l'urètre ou le col vésical, peut comprendre une manchette occlusive entourant ledit conduit anatomique et couplée fluidiquement à un réservoir de fluide. Une pompe permet de transférer du fluide du réservoir vers la manchette, de sorte à augmenter la compression exercée par la manchette sur le conduit, ou de la manchette vers le réservoir, de sorte à réduire la compression exercée par la manchette sur le conduit.

D'autres applications impliquant un réservoir de fluide dans un dispositif médical implanté dans le corps d'un patient concernent le gonflage d'un ballon d'obturation, ou encore le remplissage d'une prothèse.

En général, le dispositif médical adapté pour être implanté est fourni au chirurgien vide de tout fluide biocompatible, dans un emballage dont le contenu est stérile. Le chirurgien doit donc chasser l'air et remplir le réservoir d'un fluide biocompatible avant l'implantation chez le patient.

Cette opération de remplissage doit être mise en oeuvre de manière à purger le circuit fluidique de l'air contenu initialement. En effet, d'éventuelles bulles d'air sont susceptibles d'affecter le fonctionnement du dispositif implantable et en particulier sa précision.

Une autre contrainte de l'opération de remplissage est le maintien de la stérilité du dispositif.

Un remplissage manuel du dispositif par le chirurgien allonge la durée de l'intervention chirurgicale et induit un risque de contamination du dispositif, susceptible d'induire des infections ou des complications chez le patient.

Il existe des dispositifs médicaux préremplis mais ces derniers présentent de nombreux désavantages comme par exemple lors de l'étape de stérilisation pendant laquelle il faut s'assurer que le fluide est également stérilisé à travers le dispositif.

Le document US 2015/021221 A1 décrit un emballage pour un dispositif médical comprenant un réservoir de fluide destiné à être rempli après son déballage. L'emballage est formé de deux parties, l'une maintenant le dispositif médical pendant son transport et l'autre maintenant le dispositif médical pendant son remplissage.

Les documents US 2017/056149 A1 et US 2012/103840 A1 décrivent des emballages pour valves cardiaques, qui ne contiennent pas de réservoir de fluide à remplir avant implantation.

Le document US 4,726,404 A décrit un emballage pour un dispositif médical comprenant un réservoir de fluide, dont la purge et le remplissage sont réalisés manuellement par un utilisateur manipulant le dispositif.

### Brève description de l'invention

Un but de l'invention est donc de définir un dispositif médical adapté pour être implanté comprenant un réservoir de fluide et un emballage dudit dispositif, qui ensemble permettent de minimiser la manipulation du dispositif avant l'implantation.

A cet effet, l'invention concerne un ensemble formé d'un dispositif médical adapté pour être implanté dans un corps humain ou animal et d'un emballage, tel que défini dans la revendication 1, dans lequel :
- ledit dispositif médical comprend un boîtier renfermant un réservoir de fluide et une sortie fluidique formant une liaison fluidique entre le réservoir et un volume extérieur au boîtier,
- l'emballage inclut une cuve comprenant un fond, une paroi latérale, une face supérieure opposée au fond selon une direction verticale et une ouverture configurée pour remplir la cuve avec un fluide biocompatible, le fond de la cuve s'étendant dans un plan horizontal perpendiculaire à la direction verticale, l'un au moins du fond, de la paroi latérale et de la face supérieure comprend un premier élément de maintien adapté pour maintenir le boîtier dans la cuve,
le boîtier étant maintenu dans la cuve par ledit premier élément de maintien de telle sorte que le réservoir soit positionné entre le fond de la cuve et la sortie fluidique, ladite sortie fluidique étant agencée en-dessous de l'ouverture de sorte que, lorsque la cuve est remplie du fluide biocompatible, ladite sortie fluidique est immergée dans le fluide.

Un tel ensemble présente l'avantage de procurer un positionnement du dispositif médical favorable à la purge, la sortie fluidique étant située au-dessus du réservoir lorsque l'emballage est dans sa position normale d'utilisation dans laquelle le fond de la cuve repose sur une surface horizontale, telle qu'une table. Ainsi, une fois la cuve remplie d'un fluide biocompatible, la purge peut être mise en oeuvre sans aucune manipulation du dispositif.

Par « verticale » on entend dans le présent texte une direction parallèle à la direction de la pesanteur, telle qu'indiquée par exemple par un fil à plomb, et par « horizontale » une direction contenue dans un plan perpendiculaire à ladite direction verticale. Par ailleurs, les termes « au-dessus » ou « en-dessous » ou « supérieur » ou « inférieur » s'entendent par rapport à cette direction verticale.

Dans certains modes de réalisation, ledit dispositif médical comprend une tubulure en liaison fluidique avec la sortie fluidique, le fond et/ou la paroi latérale de la cuve comprenant un second élément de maintien adapté pour maintenir une portion de ladite tubulure.

Dans certains modes de réalisation, l'ouverture est agencée dans la face supérieure de la cuve. De manière alternative, l'ouverture est agencée dans une portion supérieure de la paroi latérale.

Dans certains modes de réalisation, l'emballage comprend en outre un couvercle amovible vis-à-vis de la cuve de sorte à obturer au moins partiellement l'ouverture.

Ledit couvercle peut être configuré pour être solidarisé à une partie seulement de la dite au moins une paroi latérale, le couvercle présentant au moins une zone de préhension distante de la dite au moins une paroi latérale.

Dans certains modes de réalisation, le couvercle présente en outre un troisième élément de maintien adapté pour maintenir le boîtier dans la position dans laquelle le réservoir est positionné entre le fond de la cuve et la sortie fluidique.

Chaque élément de maintien de la cuve et, le cas échéant, du couvercle, peut se présenter sous la forme d'un logement adapté pour maintenir le boîtier et/ou, le cas échéant, la tubulure, par friction.

Dans certains modes de réalisation, la cuve est formée par moulage d'un matériau plastique et chaque logement peut alors être formé lors du moulage.

Dans certains modes de réalisation, la cuve comprend un indicateur d'horizontalité du fond de la cuve.

Ledit indicateur peut être un indicateur d'horizontalité d'un niveau de fluide dans la cuve.

Dans certains modes de réalisation, l'emballage comprend en outre un bac adapté pour contenir la cuve.

Dans certains modes de réalisation, ledit ensemble comprend en outre un premier opercule perméable à un gaz stérilisant solidaire de ladite au moins une paroi latérale, la cuve et le premier opercule formant ensemble une enceinte stérile pour le dispositif médical.

Ledit ensemble peut comprendre en outre un second opercule perméable à un gaz stérilisant obturant le bac, le bac et le second opercule formant ensemble une enceinte stérile pour la cuve.

Dans certains modes de réalisation, le réservoir comprend une paroi fixe et une paroi mobile par rapport à la partie fixe pour faire varier le volume du réservoir, ladite variation de volume du réservoir étant adaptée pour le remplissage par un fluide biocompatible et la purge d'un circuit fluidique s'étendant entre le réservoir et la sortie fluidique et ce, de manière automatique.

De manière avantageuse, le dispositif médical comprend un actionneur électromécanique agencé dans le boîtier, ledit actionneur étant configuré pour déplacer la paroi mobile du réservoir pour faire varier le volume du réservoir.

Dans certains modes de réalisation, le boîtier comprend une unique sortie fluidique et une unique liaison fluidique entre le réservoir et ladite sortie fluidique.

Dans certains modes de réalisation, le dispositif médical est choisi dans le groupe consistant en un sphincter artificiel, un muscle artificiel, un stimulateur électrique, un anneau gastrique, un neurostimulateur et un implant pénien.

### Brève description des dessins

D'autres caractéristiques et avantages de l'invention ressortiront de la description détaillée qui va suivre, en référence aux dessins annexés, sur lesquels :
- la figure 1 est une vue en coupe du dispositif médical selon un mode de réalisation ;
- la figure 2 est une vue éclatée d'un emballage selon un mode de réalisation ;
- la figure 3 est une vue en perspective de la cuve ;
- la figure 4 est une vue en coupe de l'emballage assemblé de la figure 2 ;
- la figure 5 est une vue en perspective du dispositif médical dans son emballage.

### Description détaillée de modes de réalisation

Le dispositif médical comprend un boîtier en un matériau biocompatible, par exemple du titane.

Le boîtier contient un réservoir de fluide biocompatible et est pourvu d'une sortie fluidique qui forme une liaison fluidique entre le réservoir et le milieu extérieur au dispositif médical. Ladite sortie fluidique peut notamment être connectée à une tubulure assurant une liaison fluidique avec un élément extérieur au boîtier. De préférence, ledit fluide biocompatible est un liquide.

Selon un mode de réalisation, le réservoir comprend une paroi mobile, mue par un actionneur, dont le déplacement permet de faire varier le volume du réservoir de manière contrôlée. Cette variation de volume permet de déplacer du fluide du réservoir vers la sortie fluidique ou de la sortie fluidique vers le réservoir.

De manière particulièrement avantageuse, l'actionneur est un actionneur électromécanique, qui est commandé par une unité de commande pour déplacer la paroi mobile d'une distance déterminée. L'unité de commande peut être agencée dans le boîtier. Le dispositif médical est ainsi autonome, en ce sens qu'il ne nécessite pas d'action du praticien pour remplir sa fonction.

Par ailleurs, le dispositif comprend avantageusement une unique sortie fluidique et un unique circuit fluidique entre le réservoir et la sortie fluidique.

Dans certaines applications, le dispositif médical est un sphincter artificiel, destiné à obturer sélectivement un conduit anatomique en exerçant un effort de compression sur ledit conduit. En particulier, le dispositif médical peut être un sphincter urinaire artificiel.

Dans un tel dispositif, la compression est exercée sur le conduit anatomique au moyen d'une manchette occlusive reliée à la sortie fluidique du boîtier par une tubulure. L'actionneur électromécanique, en déplaçant la paroi mobile, permet de transférer du fluide du réservoir vers la manchette ou de la manchette vers le réservoir, afin d'ajuster la compression exercée par la manchette.

Des exemples d'un tel dispositif médical sont décrits dans le document WO 2016/083428.

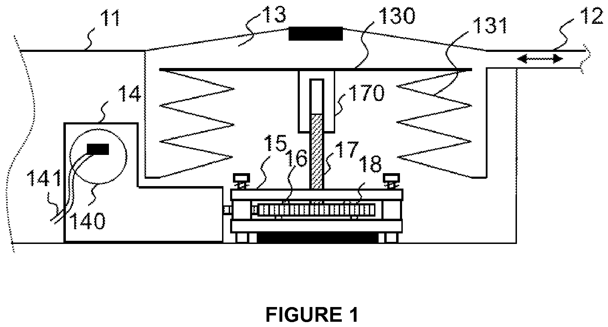

La figure 1 illustre un mode de réalisation du dispositif médical.

Le dispositif médical comprend :
- un boîtier étanche 11 contenant un gaz,
- une sortie fluidique 12,
- un réservoir de fluide 13 présentant un volume de fluide variable agencé dans le boîtier en liaison fluidique avec la sortie fluidique 12.

De manière particulièrement avantageuse, le réservoir comprend une paroi fixe et une paroi mobile par rapport à la paroi fixe, un déplacement de ladite paroi mobile permettant de faire varier le volume du réservoir 13.

Selon un mode de réalisation avantageux mais non limitatif, la paroi fixe du réservoir peut être formée en partie de la paroi intérieure du boîtier 11, et la paroi mobile 130, qui est de préférence rigide, est reliée à la paroi fixe par un soufflet 131 déformable.

Un actionneur 14 est agencé dans le boîtier 11 et couplé mécaniquement à la paroi mobile 130 pour faire varier sélectivement le volume de fluide dans ledit réservoir.

L'actionneur 14 peut être adapté pour commander un déplacement linéaire de la paroi mobile 130, le soufflet 131 étant adapté pour s'étendre ou se comprimer en fonction dudit déplacement linéaire de la paroi mobile 130 commandé par l'actionneur 14.

L'actionneur 14 peut être choisi parmi tout système électromécanique permettant de transformer une énergie électrique en un mouvement mécanique avec la puissance requise pour permettre le déplacement à une force et à une vitesse requise de la paroi mobile 130 du réservoir 13 à volume variable. L'actionneur 14 peut notamment être un actionneur piézo-électrique, un actionneur électromagnétique pouvant comprendre un moteur électromagnétique avec ou sans balai couplé ou non à un réducteur, un polymère électro-actif ou un alliage à mémoire de forme.

La paroi mobile 130 peut être déplacée en translation suivant un axe longitudinal par l'action d'une vis d'entraînement 17 solidaire de la paroi mobile 130. La paroi mobile 130 est couplée à la vis d'entraînement 17 par l'intermédiaire d'une noix 170 solidaire de la paroi mobile 130 et présentant un taraudage coopérant avec le filetage de la vis 17. La vis d'entraînement 17 peut s'étendre sensiblement suivant l'axe longitudinal. Une position de la vis d'entraînement 17 peut correspondre sensiblement à un centre de la paroi mobile 130.

L'actionneur 14 est adapté pour entraîner la vis d'entraînement 17 en rotation, par exemple par le biais de la rotation d'un pignon. Une rotation de la vis d'entraînement 17 autour de l'axe longitudinal entraîne un déplacement de la paroi mobile 130 le long de l'axe longitudinal, le soufflet 131 se comprimant ou s'étendant le long de l'axe longitudinal en conséquence.

L'actionneur 14 peut comprendre un moteur 140 couplé à un réducteur. Un connecteur 141 permet d'alimenter le moteur 140 en fonction d'un ordre de fonctionnement du moteur.

Le réducteur est couplé à une roue dentée 18 qui est elle-même couplée à la vis d'entraînement 17, de sorte à transmettre le couple et la rotation de l'axe du moteur 140 à la vis d'entraînement 17. Le système d'entraînement comprend donc une noix couplée à la vis d'entraînement et mobile en rotation sur une butée à billes à double effet autour de l'axe de la vis sous l'effet d'une action d'entraînement par l'actionneur 14, la noix étant couplée à la vis de sorte qu'une rotation de la noix entraîne la vis uniquement en translation dans la direction de déplacement de la partie mobile.

La rotation de la vis 17 entraîne alors la noix en translation, ce qui a pour effet de déplacer la paroi mobile 130 en translation dans une direction parallèle à l'axe de la vis, c'est-à-dire dans la direction de l'axe longitudinal. Le sens de déplacement de la paroi mobile 130 dépend du sens de rotation du moteur 140.

La roue dentée 18 est logée dans un bloc 15 par l'intermédiaire de roulements à billes 16 qui permettent sa rotation dans le bloc 15.

Naturellement, la figure 1 ne présente un mode de réalisation du dispositif médical qu'à titre d'illustration, et tout autre agencement d'un réservoir à volume variable et d'un actionneur peuvent être définis par l'homme du métier pour remplir la fonction dudit dispositif médical.

Un tel dispositif se distingue des sphincters artificiels comprenant une pompe actionnable manuellement par le patient, un ballon de régulation de pression et une manchette, par le fait que le dispositif a un fonctionnement autonome commandable, sans action manuelle répétitive du patient.

Dans d'autres applications, le dispositif médical peut être un muscle artificiel, un stimulateur électrique, un anneau gastrique, un neurostimulateur ou un implant pénien (liste non limitative).

Entre le site de fabrication du dispositif médical et le bloc opératoire dans lequel le dispositif médical est implanté chez un patient, le dispositif médical est agencé dans un emballage qui fait l'objet du présent texte. Ledit emballage est adapté pour maintenir le dispositif médical stérile en le protégeant des contaminations extérieures et pour le protéger des chocs. Dans l'emballage, le dispositif médical est vide de fluide biocompatible, le remplissage du circuit fluidique n'étant réalisé qu'au bloc opératoire.

Comme décrit par la suite, ledit emballage est en outre adapté pour permettre la purge du circuit fluidique sans manipulation du dispositif médical par un praticien au bloc opératoire.

L'emballage comprend tout d'abord une cuve dans laquelle le dispositif médical est maintenu dans une position dite verticale, c'est-à-dire dans laquelle dans le réservoir est positionné entre le fond de la cuve et la sortie fluidique.

La cuve comprend un fond horizontal, une paroi latérale, une face supérieure opposée au fond selon la direction verticale.

La cuve comprend au moins une ouverture configurée pour permettre le remplissage de la cuve avec un fluide biocompatible.

La sortie fluidique du dispositif médical est située en-dessous de ladite ouverture, de sorte que, lors du remplissage de la cuve, l'ensemble du dispositif médical, incluant la sortie fluidique, est immergé dans le fluide.

Pour s'assurer que le remplissage de la cuve en fluide biocompatible est suffisant pour mettre en oeuvre le procédé de purge, la cuve peut avantageusement comporter un indicateur visuel de niveau d'eau disposé dans la partie haute de la cuve. Avantageusement, le volume de la cuve et la position de l'indicateur ont été choisis et dimensionnés pour permettre le remplissage du réservoir du dispositif. Alternativement, la cuve peut comporter une cavité en partie supérieure destinée à recevoir un trop-plein de fluide biocompatible de sorte à réguler le débordement de ce fluide et éviter un remplissage excessif.

Le dispositif médical est maintenu dans la position verticale par un premier élément de maintien agencé sur le fond, la paroi latérale et/ou la face supérieure de la cuve.

Selon un mode de réalisation, ledit premier élément de maintien se présente sous la forme d'un logement dont les dimensions sont adaptées pour recevoir une partie du boîtier en exerçant un léger serrage pour le maintenir dans la position souhaitée, sans l'endommager.

De manière avantageuse, ledit logement est agencé dans le fond de la cuve.

Selon un mode de réalisation préféré, la cuve est réalisée par exemple par moulage ou formage d'un matériau plastique, et le logement est intégré au fond de la cuve par moulage ou formage. Le logement peut par exemple se présenter sous la forme de deux cloisons parallèles distantes de la largeur du boîtier.

Dans certains modes de réalisation, le dispositif médical peut être emballé avec une tubulure en liaison fluidique avec la sortie fluidique. Dans ce cas, la cuve comprend avantageusement un second élément de maintien agencé sur le fond et/ou la paroi latérale de la cuve pour maintenir une portion de ladite tubulure en-dessous de la sortie fluidique.

De manière avantageuse, ledit second élément de maintien se présente sous la forme d'un logement agencé dans le fond de la cuve, parallèlement au logement du boîtier. Ce logement peut par exemple se présenter sous la forme de deux cloisons parallèles distantes du diamètre de la tubulure.

Selon un mode de réalisation adapté, représenté sur les figures, la cuve présente une forme parallélépipédique comprenant une paroi latérale à quatre côtés et un fond, la face opposée au fond étant ouverte. Cependant, cette forme particulière n'est aucunement limitative.

Ainsi, selon d'autres modes de réalisation non illustrés, la cuve peut présenter toute autre forme, telle qu'une forme cylindrique par exemple, avec une paroi latérale cylindrique et un fond circulaire.

De manière particulièrement avantageuse, le fond de la cuve présente des dimensions sensiblement identiques selon deux directions orthogonales dans un plan horizontal, afin d'assurer la stabilité de la cuve, et éviter notamment tout renversement de la cuve lors de la purge du dispositif médical.

Selon certains modes de réalisation non illustrés, l'ouverture de la cuve peut ne pas être située sur la face supérieure de la cuve mais sur la paroi latérale de la cuve, de préférence dans une portion supérieure de ladite paroi de sorte à se trouver au-dessus de la sortie fluidique du dispositif médical.

Dans tous les cas, la cuve est dimensionnée et l'ouverture est agencée de sorte que, lorsque le fond de la cuve s'étend dans un plan horizontal, la cuve puisse être remplie de fluide biocompatible au moins jusqu'au niveau de la sortie fluidique du dispositif médical, de préférence au-dessus de ladite sortie. Ainsi, lors de la purge, l'ensemble du circuit fluidique s'étendant entre le réservoir et la sortie fluidique peut être rempli de fluide biocompatible et purgé de l'air présent initialement. En particulier, le remplissage et la purge peuvent être effectués au travers de ladite sortie fluidique, par variation du volume du réservoir, sans qu'une intervention d'un utilisateur autre qu'une activation de ladite variation de volume ne soit nécessaire, évitant ainsi toute contamination.

De manière avantageuse, l'emballage comprend en outre un couvercle amovible vis-à-vis de la cuve de sorte à obturer au moins partiellement l'ouverture. Le couvercle est de préférence emboîté sur la paroi latérale, et retenu sur celle-ci par friction ou par emboitement par exemple.

Selon un mode de réalisation préféré, le couvercle est configuré pour être solidarisé à une partie seulement de la paroi latérale, le couvercle présentant au moins une zone de préhension distante de la paroi latérale. Par exemple, le couvercle peut présenter un contour dont une partie coïncide avec la forme de l'ouverture définie par la paroi latérale et une partie est en retrait de la paroi latérale, pour former un passage pour les doigts du praticien facilitant la préhension du couvercle.

De manière particulièrement avantageuse, le couvercle présente un autre élément de maintien adapté pour maintenir le dispositif médical dans la position verticale. Cet élément de maintien peut se présenter sous la forme d'un logement agencé en regard du logement formé dans la cuve. Ainsi, le dispositif médical est maintenu dans la position verticale en deux endroits, ce qui améliore sa stabilité notamment pendant son transport.

Comme la cuve, le couvercle peut être réalisé par moulage ou formage d'un matériau plastique, et le logement est intégré à l'intérieur du couvercle par moulage ou formage. Le logement peut par exemple se présenter sous la forme de deux cloisons parallèles distantes de la largeur du boîtier.

Dans certains modes de réalisation, l'emballage comprend un bac destiné à contenir la cuve. Le bac permet de renforcer la protection du dispositif médical contre les chocs. Le bac forme également avantageusement une enceinte stérile pour la cuve, ce qui améliore la protection du dispositif médical contre les contaminants extérieurs. De préférence, lors de l'opération d'implantation, le bac peut être manipulé et ouvert en dehors du champ stérile du bloc opératoire de sorte à pouvoir en extraire la cuve qui est transmise dans le champ stérile. La cuve est ensuite ouverte et manipulée par le praticien dans le champ stérile. Par ailleurs, comme expliqué plus bas, le bac peut être utilisé dans le procédé de purge, en étant rempli de fluide biocompatible.

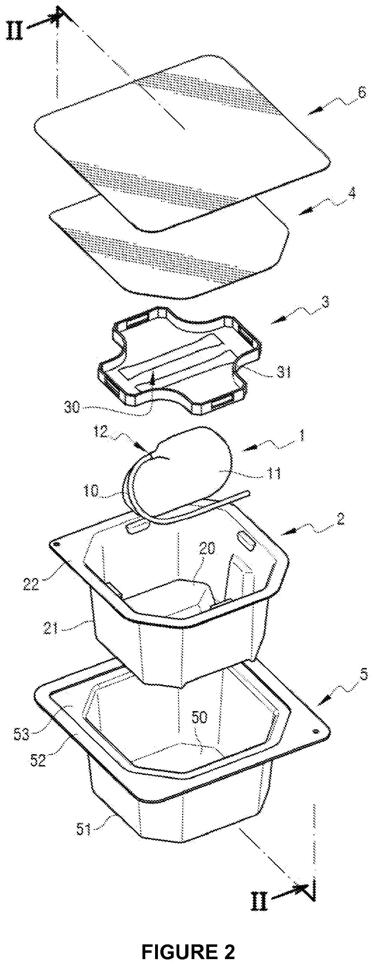

Le procédé de conditionnement du dispositif médical peut être mis en oeuvre de la façon suivante, avec l'emballage illustré sur la figure 2.

Le dispositif médical assemblé 1 est mis en place dans la cuve 2. A cet effet, on engage le dispositif médical dans le ou les élément(s) de maintien prévu(s) dans la cuve pour maintenir ledit dispositif avec la sortie fluidique au-dessus du réservoir. Lorsque la sortie fluidique est connectée à une tubulure, ladite tubulure est également de préférence engagée dans le ou les élément(s) de maintien prévu(s) dans la cuve pour maintenir ladite tubulure au fond de la cuve.

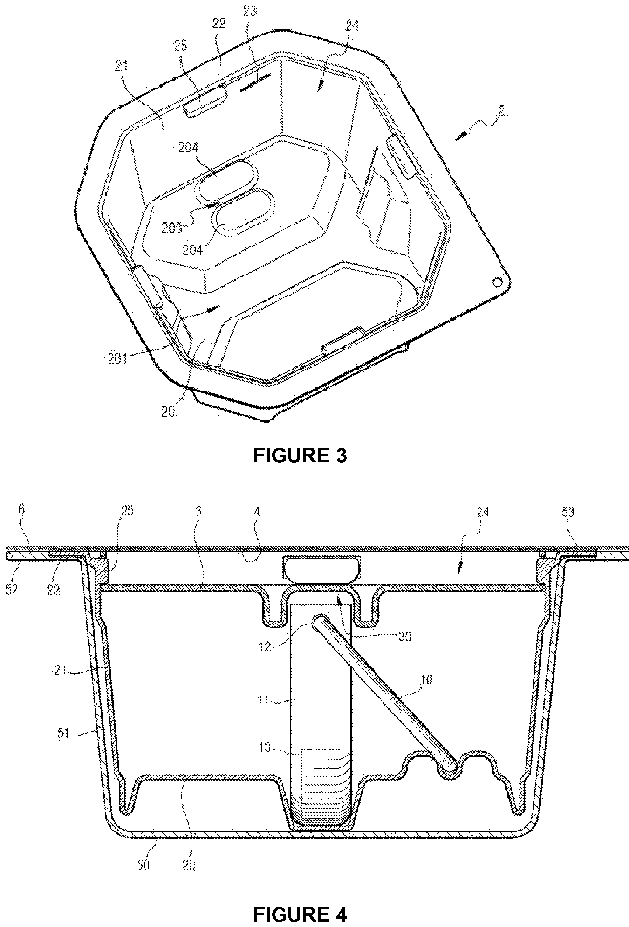

En référence à la figure 3, la cuve 2 comprend un fond 20 et une paroi latérale 21 qui s'étend sensiblement verticalement à partir du fond 20. Du côté opposé au fond 20, la paroi latérale 21 présente un bord 22 qui s'étend vers l'extérieur dans un plan horizontal. Ledit bord 22 définit une face supérieure de la cuve pourvue d'une ouverture 24.

Comme indiqué plus haut en référence à la figure 1, le dispositif médical 1 comprend un boîtier 11 dans lequel est agencé un réservoir de fluide biocompatible 13 (schématisé en pointillés sur la figure 4). Le dispositif médical 1 comprend en outre une sortie fluidique 12 qui est connectée au boîtier 11 et en liaison fluidique avec le réservoir 13. La sortie fluidique 12 assure une liaison fluidique entre le réservoir et l'extérieur du boîtier. La sortie fluidique 12 est connectée à une tubulure 10, qui est destinée à être reliée, lors de l'implantation, à la manchette occlusive.

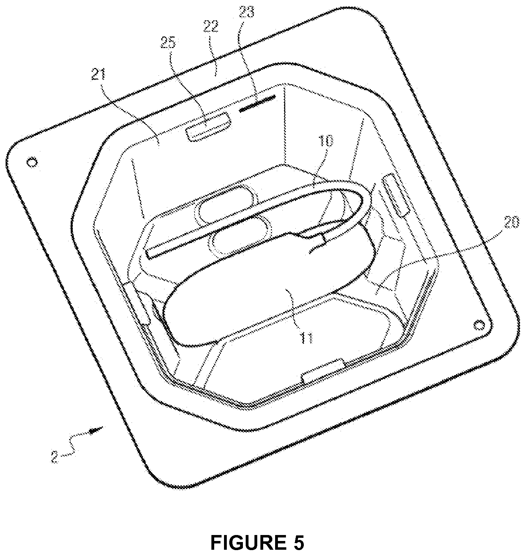

La hauteur de la paroi latérale 21 est supérieure à la hauteur du dispositif médical dans sa position verticale de conditionnement. Ainsi, lorsque le dispositif médical 1 est agencé dans la cuve 2, comme illustré sur les figures 4 et 5, le dispositif médical est entièrement contenu dans la cuve 2. La sortie fluidique 12 est agencée sous l'ouverture 24, de sorte à pouvoir être recouverte de fluide lors du remplissage de la cuve. Le réservoir 13 est agencé sous la sortie fluidique 12 lorsque le dispositif médial est disposé dans la cuve.

Le fond 20 présente une forme sensiblement carrée afin d'assurer la stabilité de la cuve.

Le fond 20 de la cuve 2 comprend un logement 201 adapté pour maintenir le dispositif médical en position verticale. Le logement 201 est défini comme une cavité formée dans le fond 20, parallèlement à l'un des côtés, et s'étend sensiblement entre le milieu d'un côté et le milieu du côté opposé du fond 20. L'emplacement du logement est dans une région centrale du fond pour assurer la stabilité de l'ensemble cuve - dispositif médical lorsque le dispositif médical est agencé dans le logement et la cuve est remplie de fluide biocompatible. La largeur dudit logement est sensiblement égale à celle du dispositif médical, afin de permettre l'insertion et le maintien du dispositif médical dans ledit logement. La profondeur du logement est choisie de sorte à éviter tout basculement du boîtier 11 ; une profondeur de 5 à 15 mm est adaptée à cette fonction.

De manière particulièrement avantageuse, le logement 201 est pourvu de deux paires de protrusions arrondies (non illustrées), dans lesquelles les protrusions de chaque paire sont agencées en vis-à-vis dans le sens de la largeur du logement 201. Chaque paire de protrusions restreint localement la largeur du logement 201 et exerce ainsi un léger serrage sur le dispositif médical pour son maintien en position.

Le fond 20 de la cuve comprend en outre un logement 203 pour la tubulure 10 du dispositif médical. La largeur dudit logement est sensiblement égale au diamètre de la tubulure. Ledit logement se présente sous la forme de deux plots 204 parallèles en relief par rapport au fond 20. De manière avantageuse, comme pour le logement 201, les plots présentent une paire de protrusions arrondies (non illustrées) en vis-à-vis dans le sens de la largeur du logement 203. Ladite paire de protrusions restreint localement la largeur du logement 203 et exerce ainsi un léger serrage sur la tubulure. Le logement 203 est avantageusement parallèle au logement 201.

De manière alternative, le logement 201 pourrait être défini par une ou plusieurs paires de plots en vis-à-vis, en relief par rapport au fond de la cuve. Le logement 203 pourrait quant à lui être défini comme une cavité dans le fond de la cuve. Toute autre forme adaptée à un maintien du dispositif médical en position verticale pourrait être utilisée.

Le couvercle 3 est mis en place sur la cuve 2. Le dispositif médical est alors engagé dans le ou le(s) élément(s) de maintien prévu(s) dans le couvercle pour maintenir ledit dispositif avec la sortie fluidique au-dessus du réservoir.

Par exemple, le couvercle peut comprendre, en regard du logement 201, un logement 30 formé dans le couvercle. Le logement 30 peut être défini par deux plots en relief par rapport à la surface intérieure du couvercle, ou comme un creux formé dans la surface intérieure du couvercle. La largeur du logement 30 est sensiblement égale à la largeur de la partie supérieure du dispositif médical 1 dans la position verticale.

De manière avantageuse, le couvercle 3 présente sensiblement une forme de croix. De ce fait, le couvercle n'obture qu'une partie de l'ouverture supérieure de la cuve 2, et laisse au niveau des coins de la cuve des ouvertures adaptées pour la préhension du couvercle au niveau de zones de préhension 31 par un praticien.

Les formes et dimensions respectives de la paroi latérale 21 et du bord du couvercle 3 sont choisies pour permettre un emboîtement du couvercle dans la cuve. En particulier, la paroi latérale 21 peut comprendre, dans sa partie supérieure, un rebord 25 adapté pour retenir un bord respectif du couvercle 3.

Une première feuille 4 perméable à un gaz stérilisant, par exemple un opercule en Tyvek^{™}, est ensuite scellée sur le bord supérieur 22 de la cuve.

La cuve 2 est ensuite mise en place dans le bac 5.

Le bac 5 présente une forme similaire à celle de la cuve 2 mais des dimensions supérieures. De préférence, le bac 5 présente une taille suffisante pour recevoir entièrement la cuve 2 tout en minimisant l'espace libre entre la cuve 2 et le bac 5, afin de minimiser l'encombrement de l'emballage.

Le bac 5 présente un fond 50 sensiblement plan et une paroi latérale qui s'étend sensiblement verticalement à partir du fond 50. Du côté opposé au fond 50, la paroi latérale 51 présente un bord 52 qui s'étend vers l'extérieur dans un plan horizontal. Ledit bord 52 définit une face supérieure du bac pourvue d'une ouverture centrale.

De manière avantageuse, le bac 5 présente un rebord intérieur 53 procurant un appui pour le bord 22 de la cuve 2.

Comme le fond 20 de la cuve, le fond 50 du bac 5 est sensiblement carré pour assurer une bonne stabilité du bac.

Une seconde feuille 6 perméable à un gaz stérilisant, par exemple un opercule en Tyvek^{™}, est ensuite scellée sur le bord supérieur 52 du bac 5.

L'emballage ainsi scellé est stérilisé par tout moyen adapté, puis conditionné dans une boîte (non illustrée) qui constitue l'enveloppe externe dans laquelle le dispositif médical est livré à un praticien pour l'implantation chez un patient.

En vue de cette implantation, le praticien sort l'emballage de la boîte, retire la seconde feuille de scellage, sort la cuve puis retire la première feuille de scellage.

Selon un mode de réalisation, le praticien sort la cuve du bac en vue de la remplir de fluide biocompatible.

Pour effectuer ce remplissage et la mise en oeuvre de la purge, le praticien doit s'assurer que le fond de la cuve soit horizontal.

L'horizontalité du fond de la cuve peut être assurée en posant le fond de la cuve sur une surface plane et horizontale, par exemple une table.

Cette horizontalité peut être confirmée au moyen d'un indicateur adéquat. Par exemple, un tel indicateur d'horizontalité peut être un indicateur de l'horizontalité d'un niveau d'eau dans la cuve. Cet indicateur peut par exemple se présenter sous la forme d'un ou plusieurs segments formés dans la cuve, par exemple directement par moulage, voire par impression, et placés dans la partie supérieure de la cuve, parallèlement au fond. Un tel indicateur peut également servir d'indicateur de remplissage pour le praticien, lui signalant jusqu'à quelle hauteur de la cuve il doit verser le fluide biocompatible pour permettre une purge efficace. La figure 3 illustre un mode de réalisation d'un tel indicateur d'horizontalité sous la forme d'une ligne intérieure 23 en creux ou en relief s'étendant dans un plan horizontal à l'intérieur de la paroi latérale 21. De préférence, ladite ligne intérieure 23 s'étend sur plusieurs côtés de la paroi latérale 21, de manière continue ou discontinue.

Une indication sur l'horizontalité du fond de la cuve peut également être fournie par le dispositif médical lui-même. En effet, le dispositif comprend généralement un accéléromètre agencé dans le boîtier, qui peut être utilisé, lorsque le dispositif médical est implanté, pour déterminer une posture ou une activité du patient. Le boîtier étant solidaire du fond de la cuve avec une orientation déterminée par rapport à celui-ci (généralement perpendiculaire), l'accéléromètre peut fournir une indication de l'inclinaison du fond de la cuve.

De manière alternative ou complémentaire, cette horizontalité peut être assurée en plaçant la cuve dans un récipient rempli lui-même d'un fluide. Dans ce cas, la cuve est dimensionnée pour que, lorsqu'elle contient le dispositif médical dans la position prévue pour la purge, elle flotte dans le récipient en maintenant la sortie fluidique du dispositif médical au-dessus du réservoir.

Le bac dans lequel la cuve est agencée dans l'emballage peut être utilisé en tant que récipient à cet effet.

Le praticien remplit alors la cuve avec du fluide biocompatible qui est destiné au fonctionnement du dispositif médical. Ledit fluide peut être par exemple du sérum physiologique.

Il active ensuite l'actionneur du dispositif médical pour chasser l'air présent dans le circuit fluidique s'étendant entre le réservoir et la sortie fluidique et dans la tubulure le cas échéant, et remplir ledit circuit fluidique et la tubulure avec le fluide biocompatible. L'actionneur peut être activé plusieurs fois, par exemple trois fois, entrainant le déplacement d'une paroi mobile du réservoir pour faire varier un volume du réservoir et ainsi exercer une action de pompage, jusqu'à ce que le circuit fluidique soit entièrement purgé (aucune bulle d'air ne s'échappant de la sortie fluidique).

Cette activation de l'actionneur ne nécessite pas de manipulation du dispositif médical lui-même. En effet, le dispositif médical comprend des moyens de communication sans fil avec une télécommande externe, qui permet de paramétrer le dispositif et de lui envoyer des ordres d'activation ou de désactivation.

Une fois la purge achevée, le praticien peut obturer la sortie fluidique ou l'extrémité de la tubulure le cas échéant, et sort le dispositif médical de la cuve en vue de l'opération d'implantation.

Ainsi, avant l'implantation, le praticien n'a pas touché le dispositif médical, ce qui permet d'éviter ou tout au moins de minimiser les risques de contamination du dispositif.

Par ailleurs, l'intervention du praticien n'est pas requise pendant la mise en oeuvre de la purge. En effet, une fois que la purge a été enclenchée, c'est l'actionneur du dispositif qui aspire le fluide biocompatible et chasse l'air. Le praticien peut donc se consacrer à d'autres tâches, telles que la préparation de l'implantation. Le temps chirurgical peut ainsi être réduit de manière significative.

Enfin, le fait que la mise en oeuvre de la purge soit automatique accroit la fiabilité du procédé.

Il est entendu que les modes de réalisation décrits ci-dessus correspondent à des exemples particuliers et non-limitatifs et que diverses modifications peuvent être apportées sans pour autant s'éloigner de l'invention.

## Revendications

1. Ensemble formé d'un dispositif médical (1) adapté pour être implanté dans un corps humain ou animal et d'un emballage, dans lequel :
- ledit dispositif médical comprend un boîtier (11) renfermant un réservoir de fluide (13) et une sortie fluidique (12) formant une liaison fluidique entre le réservoir et un volume extérieur au boîtier,
- l'emballage inclut une cuve (2) comprenant un fond (20), une paroi latérale (21) et une face supérieure opposée au fond selon une direction verticale, le fond (20) de la cuve s'étendant dans un plan horizontal perpendiculaire à la direction verticale, l'un au moins du fond, de la paroi latérale et de la face supérieure comprend un premier élément de maintien (201) adapté pour maintenir le boîtier dans la cuve,
**caractérisé en ce que** la cuve comprend en outre une ouverture configurée pour remplir la cuve avec un fluide biocompatible de sorte à remplir dudit fluide biocompatible un circuit fluidique s'étendant de la sortie fluidique au réservoir du dispositif médical,
le boîtier (11) étant maintenu dans la cuve (2) par ledit premier élément de maintien (201) de telle sorte que le réservoir soit positionné entre le fond de la cuve et la sortie fluidique pour permettre de purger le circuit fluidique sans manipulation du dispositif médical, ladite sortie fluidique étant agencée en-dessous de l'ouverture de sorte que, lorsque la cuve est remplie du fluide biocompatible, ladite sortie fluidique est immergée dans le fluide.

2. Ensemble selon la revendication 1, dans lequel ledit dispositif médical (1) comprend une tubulure (10) en liaison fluidique avec la sortie fluidique (12), le fond et/ou la paroi latérale de la cuve comprenant un second élément de maintien (203) adapté pour maintenir une portion de ladite tubulure.

3. Ensemble selon l'une des revendications 1 ou 2, dans lequel l'ouverture est agencée dans la face supérieure de la cuve.

4. Ensemble selon l'une des revendications 1 ou 2, dans lequel l'ouverture est agencée dans une portion supérieure de la paroi latérale.

5. Ensemble selon l'une des revendications 1 ou 2, comprenant en outre un couvercle (3) amovible vis-à-vis de la cuve (2) de sorte à obturer au moins partiellement l'ouverture.

6. Ensemble selon la revendication 5, dans lequel le couvercle (3) est configuré pour être solidarisé à une partie seulement de la dite au moins une paroi latérale (21), le couvercle (3) présentant au moins une zone de préhension (31) distante de la dite au moins une paroi latérale.

7. Ensemble selon l'une des revendications 5 à 6, dans lequel le couvercle (3) présente en outre un troisième élément de maintien (30) adapté pour maintenir le boîtier dans la position dans laquelle le réservoir est positionné entre le fond de la cuve et la sortie fluidique.

8. Ensemble selon l'une des revendications 1 à 7, dans lequel chaque élément de maintien (201, 203, 30) est un logement adapté pour maintenir le boîtier (11) et/ou, le cas échéant, la tubulure (10), par friction.

9. Ensemble selon la revendication 8, dans lequel la cuve (2) est formée par moulage d'un matériau plastique et dans lequel chaque logement (201, 203, 30) est venu de moulage.

10. Ensemble selon l'une des revendications 1 à 9, dans lequel la cuve (2) comprend un indicateur d'horizontalité du fond de la cuve.

11. Ensemble selon la revendication 10, dans lequel l'indicateur d'horizontalité du fond de la cuve est un indicateur (23) d'horizontalité d'un niveau de fluide dans la cuve.

12. Ensemble selon l'une des revendications 1 à 11, comprenant en outre un bac (5) adapté pour contenir la cuve (2).

13. Ensemble selon l'une des revendications 1 à 12, comprenant en outre un premier opercule (4) perméable à un gaz stérilisant solidaire de ladite au moins une paroi latérale (21), la cuve (2) et le premier opercule (4) formant ensemble une enceinte stérile pour le dispositif médical (1).

14. Ensemble selon la revendication 13 en combinaison avec la revendication 12, comprenant en outre un second opercule (6) perméable à un gaz stérilisant obturant le bac (5), le bac (5) et le second opercule (6) formant ensemble une enceinte stérile pour la cuve (2).

15. Ensemble selon l'une des revendications 1 à 14, dans laquelle le réservoir (13) comprend une paroi fixe et une paroi (130) mobile par rapport à la partie fixe pour faire varier le volume du réservoir, ladite variation de volume du réservoir étant adaptée pour le remplissage par le fluide biocompatible et la purge du circuit fluidique.

16. Ensemble selon la revendication 15, dans lequel le dispositif médical (1) comprend un actionneur électromécanique (14) agencé dans le boîtier, ledit actionneur étant configuré pour déplacer la paroi mobile (130) du réservoir de sorte à faire varier le volume du réservoir.

17. Ensemble selon l'une des revendications 1 à 16, dans lequel le boîtier (11) comprend une unique sortie fluidique (12) et une unique liaison fluidique entre le réservoir (13) et ladite sortie fluidique (12).

18. Ensemble selon l'une des revendications 1 à 17, dans lequel le dispositif médical (1) est choisi dans le groupe consistant en un sphincter artificiel, un muscle artificiel, un stimulateur électrique, un anneau gastrique, un neurostimulateur et un implant pénien.

## Patentansprüche

1. Baugruppe aus einer medizinischen Vorrichtung (1), das für die Implantation in einen menschlichen oder tierischen Körper geeignet ist, und einer Verpackung:
- die medizinische Vorrichtung umfasst ein Gehäuse (11), das einen Fluidbehälter (13) und einen Fluidausgang (12) enthält, der eine Fluidverbindung zwischen dem Behälter und einem Volumen außerhalb des Gehäuses bildet,
- die Verpackung beinhaltet ein Becken (2), das einen Boden (20), eine Seitenwand (21) und eine dem Boden gegenüberliegende Oberseite in vertikaler Richtung umfasst, wobei sich der Boden (20) des Beckens in einer horizontalen Ebene senkrecht zur vertikalen Richtung erstreckt, wobei wenigstens eines des Bodens, der Seitenwand und der Oberseite ein erstes Halteelement (201) umfasst, das geeignet ist, das Gehäuse im Becken zu halten, **dadurch gekennzeichnet, dass** das Becken ferner eine Öffnung aufweist, die so eingerichtet ist, um das Becken mit einem biokompatiblen Fluid zu füllen, sodass ein Fluidkreislauf, der sich vom Fluidausgang zum Behälter der medizinischen Vorrichtung erstreckt, mit diesem biokompatiblen Fluid gefüllt wird,
wobei das Gehäuse (11) durch das erste Halteelement (201) so in dem Becken (2) gehalten wird, dass der Behälter zwischen dem Beckenboden und dem Fluidausgang positioniert ist, um das Entleeren des Fluidkreislaufs ohne Manipulation der medizinischen Vorrichtung zu ermöglichen, wobei der Fluidausgang unterhalb der Öffnung angeordnet ist, sodass, wenn das Becken mit biokompatiblem Fluid gefüllt ist, der Fluidausgang in das Fluid eingetaucht ist.

2. Baugruppe nach Anspruch 1, wobei die medizinische Vorrichtung (1) ein Rohr (10) umfasst, das mit dem Fluidausgang (12) fluidisch verbunden ist, wobei der Boden und/oder die Seitenwand des Behälters ein zweites Halteelement (203) umfasst, das geeignet ist, einen Teil des Rohrs zu halten.

3. Baugruppe nach einem der Ansprüche 1 oder 2, wobei die Öffnung in der Oberseite des Beckens angeordnet ist.

4. Baugruppe nach einem der Ansprüche 1 oder 2, wobei die Öffnung in einem oberen Teil der Seitenwand angeordnet ist.

5. Baugruppe nach einem der Ansprüche 1 oder 2, ferner umfassend einen beweglichen Deckel (3) gegenüber dem Becken (2), sodass die Öffnung zumindest teilweise verschlossen wird.

6. Baugruppe nach Anspruch 5, wobei der Deckel (3) so eingerichtet ist, dass er nur mit einem Teil der wenigstens einen Seitenwand (21) verbunden ist, wobei der Deckel (3) wenigstens einen von der wenigstens einen Seitenwand entfernten Griffbereich (31) aufweist.

7. Baugruppe nach einem der Ansprüche 5 bis 6, wobei der Deckel (3) ferner ein drittes Halteelement (30) aufweist, das geeignet ist, das Gehäuse in der Position zu halten, in der der Behälter zwischen dem Boden des Behälters und dem fluidischen Ausgang positioniert ist.

8. Baugruppe nach einem der Ansprüche 1 bis 7, wobei jedes Halteelement (201, 203, 30) eine Aufnahme ist, die geeignet ist, um das Gehäuse (11) und/oder gegebenenfalls das Rohr (10) durch Reibung zu halten.

9. Baugruppe nach Anspruch 8, wobei das Becken (2) durch Gießen eines Kunststoffmaterials geformt wird und wobei jede Aufnahme (201, 203, 30) aus dem Gießen stammt.

10. Baugruppe nach einem der Ansprüche 1 bis 9, wobei das Becken (2) eine Horizontalitätsanzeige des Beckenbodens umfasst.

11. Baugruppe nach Anspruch 10, wobei die Horizontalitätsanzeige des Beckenbodens eine Horizontalitätsanzeige (23) eines Fluidstands im Becken ist.

12. Baugruppe nach einem der Ansprüche 1 bis 11, ferner umfassend eine Wanne (5), die geeignet ist, das Becken (2) aufzunehmen.

13. Baugruppe nach einem der Ansprüche 1 bis 12, ferner umfassend einen ersten Verschluss (4), der durchlässig für ein sterilisierendes Gas ist, der mit wenigstens einer Seitenwand (21) verbunden ist, wobei das Becken (2) und der erste Verschluss (4) zusammen eine sterile Einhausung für die medizinische Vorrichtung (1) bilden.

14. Baugruppe nach Anspruch 13 in Kombination mit Anspruch 12, ferner umfassend einen zweiten Deckel (6), der durchlässig für ein sterilisierendes Gas ist, das die Wanne (5) verschließt, wobei die Wanne (5) und der zweite Deckel (6) zusammen eine sterile Einhausung für das Becken (2) bilden.

15. Baugruppe nach einem der Ansprüche 1 bis 14, wobei der Behälter (13) eine feste Wand und eine in Bezug auf den festen Teil bewegliche Wand (130) umfasst, um das Volumen des Behälters zu variieren, wobei die Volumenänderung des Behälters für das Befüllen mit dem biokompatiblen Fluid und das Entlüften des Fluidkreislaufs geeignet ist.

16. Baugruppe nach Anspruch 15, wobei die medizinische Vorrichtung (1) ein im Gehäuse angeordnetes elektromechanisches Stellglied (14) umfasst, wobei das Stellglied so eingerichtet ist, dass es die bewegliche Wand (130) des Behälters so bewegt, dass das Volumen des Behälters variiert.

17. Baugruppe nach einem der Ansprüche 1 bis 16, wobei das Gehäuse (11) einen einzigen fluidischen Ausgang (12) und eine einzige fluidische Verbindung zwischen dem Behälter (13) und dem fluidischen Ausgang (12) umfasst.

18. Baugruppe nach einem der Ansprüche 1 bis 17, wobei die medizinische Vorrichtung (1) aus der Gruppe ausgewählt ist, bestehend aus einem künstlichen Schließmuskel, einem künstlichen Muskel, einem elektrischen Stimulator, einem Magenband, einem Neurostimulator und einem Penisimplantat.

## Claims

1. Assembly of a medical device (1) adapted to be implanted in a human or animal body and a packaging, wherein:
- said medical device comprises a casing (11) enclosing a fluid reservoir (13) and a fluid outlet (12) forming a fluid link between the reservoir and a volume outside the casing,
- the packaging includes a tank (2) comprising a bottom (20), a side wall (21) and a top face opposite the bottom in a vertical direction, the bottom (20) of the tank extending in a horizontal plane perpendicular to the vertical direction, at least one of the bottom the side wall and the top face comprises a first holding element (201) adapted to hold the casing in the tank, **characterised in that** the tank further comprises an opening configured to fill the tank with a biocompatible fluid so as to fill with said biocompatible fluid a fluid circuit extending from the fluid outlet to the reservoir of the medical device ,
the casing (11) being held in the tank (2) by said first holding element (201) so that the reservoir is positioned between the bottom of the tank and the fluid outlet to allow the fluid circuit to be drained without manipulation of the medical device , said fluid outlet being arranged below the opening so that, when the tank is filled with the biocompatible fluid, said fluid outlet is immersed in the fluid .

2. Assembly according to claim 1, wherein said medical device (1) comprises a tubing (10) in fluid connection with the fluid outlet (12), the bottom and/or the side wall of the tank comprising a second holding element (203) adapted to hold a portion of said tubing.

3. Assembly according to one of claims 1 or 2, wherein the opening is arranged in the upper face of the tank.

4. Assembly according to one of claims 1 or 2, wherein the opening is arranged in an upper portion of the side wall.

5. Assembly according to one of claims 1 or 2, further comprising a lid (3) removable from the tank (2) so as to at least partially close off the opening.

6. Assembly according to claim 5, wherein the lid (3) is configured to be secured to a portion only of said at least one side wall (21), the lid (3) having at least one gripping area (31) remote from said at least one side wall.

7. Assembly according to one of claims 5 to 6, wherein the lid (3) further has a third holding element (30) adapted to hold the casing in the position in which the tank is positioned between the bottom of the tank and the fluid outlet.

8. Assembly according to one of claims 1 to 7, wherein each holding element (201, 203, 30) is a housing adapted to hold the casing (11) and/or, where appropriate, the tubing (10), by friction.

9. Assembly according to claim 8, wherein the tank (2) is formed by moulding a plastics material and wherein each housing (201, 203, 30) is moulded.

10. Assembly according to one of claims 1 to 9, wherein the tank (2) comprises a tank bottom horizontality indicator.

11. Assembly according to claim 10, wherein the tank bottom horizontality indicator is an indicator (23) of the horizontality of a fluid level in the tank.

12. Assembly according to one of claims 1 to 11, further comprising a tray (5) adapted to contain the tank (2).

13. Assembly according to one of claims 1 to 12, further comprising a first cover (4) permeable to a sterilising gas integral with said at least one side wall (21), the container (2) and the first cover (4) together forming a sterile enclosure for the medical device (1).

14. Assembly according to claim 13 in combination with claim 12, further comprising a second cover (6) permeable to a sterilising gas and sealing the tray (5), the tray (5) and the second cover (6) together forming a sterile enclosure for the tank (2).

15. Assembly according to one of claims 1 to 14, wherein the reservoir (13) comprises a fixed wall and a wall (130) which is movable relative to the fixed part in order to vary the volume of the reservoir, said variation in the volume of the reservoir being adapted for filling with biocompatible fluid and purging of the fluid circuit.

16. Assembly according to claim 15, wherein the medical device (1) comprises an electromechanical actuator (14) arranged in the casing, said actuator being configured to move the movable wall (130) of the reservoir so as to vary the volume of the reservoir.

17. Assembly according to one of claims 1 to 16, wherein the casing (11) comprises a single fluid outlet (12) and a single fluid connection between the reservoir (13) and the said fluid outlet (12).

18. Assembly according to one of claims 1 to 17, wherein the medical device (1) is selected from the group consisting of an artificial sphincter, an artificial muscle, an electrical stimulator, a gastric ring, a neurostimulator and a penile implant.
